# EUROPEAN PATENT APPLICATION

(11) **EP 4 775 237 A1**
(43) Date of publication of application: **15.07.2026**
(21) Application number: 25151717.3
(22) Date of filing: 14.01.2025
(51) Int. Cl.: A61M 5/142, A61M 5/145, A61M 5/24

(54) **A CARTRIDGE HOLDER FOR A MEDICAMENT DELIVERY DEVICE**

(71) Applicant: Ypsomed AG, 3401 Burgdorf (CH)
(72) Inventor: Binggeli, Nicolas, 3415 Rüegsauschachen (CH); Marbet, Regina, 4933 Rütschelen (CH); Münger, Manuel, 4632 Trimbach (CH); Zahno, Bernard, 3185 Schmitten (CH)

(57) **Abstract**

The present invention is concerned with a subassembly for an injection device adapted to hold or carry a medicament cartridge in a way that minimizes or eliminates breakage or cracking of said cartridge. The subassembly comprises at least two elastic walls and a recess in the housing that allows a braking motion of the cartridge. Advantages include a compact form factor and a minimum of complexity and materials required.

## Description

### FIELD OF THE INVENTION

The present invention relates to medicament delivery devices, particularly to patch injection or infusion devices which are adapted for dispensing a liquid, dissolved or suspended medicament from a glass cartridge through an injection needle or a canula into the body of a patient. More specifically, the invention pertains to a novel shock absorbing subassembly for carrying and securing the vitreous cartridge against breakage in case of impacts.

### BACKGROUND OF THE INVENTION

Medicament delivery devices, such as patch injection or infusion devices often come with a glass cartridge containing the liquid medication or substance to be administered. The use of glass cartridges brings many advantages such as easy sterilization and streamlined manufacturing. However, due to the brittle nature of glass the cartridges are susceptible to breakages or cracks in case of high force impacts such as in the case of shocks or drops. Ideally, the glass cartridges are thus mounted or fixed in the device in a way that prevents high force peaks on small areas of the cartridge in case of impacts and are surrounded by structures that can absorb impact forces so that breakage can be prevented and cartridge closure integrity (CCI) and, consequently, sterility can be maintained.

To this end a variety of shock absorbing or dampening elements for cartridge fixation are known from prior art.

US20170239427A1 discloses a shock absorbing member for an injection device comprising a sleeve configured to receive a medicament chamber wherein the sleeve includes a compressible element between the proximal and the distal end. Upon impact, the compressible element can deform and reduce its length, thus protecting the medicament chamber from shock during transport or from inadvertent damage during use or storage.

In WO2017219158A1, a method for fixating a medicament cartridge into a cartridge holder of an injection device is disclosed. The method comprises placing, inside the cartridge holder, an elastic holding element proximally of the cartridge. In case of drops, the elastic holding element can absorb kinetic energy and protect the cartridge.

WO14195183A1 discloses an injection device with a carrier that holds the needle and reservoir. The device comprises an impact damper between the housing and carrier, which can dampen axial forces on the carrier in case of drops or sudden impacts.

WO15110533A2 discloses an autoinjector comprising a cassette unit holding a medicament syringe and a damping element arranged between the syringe flange and the cassette, the damping element adopted to dampen movement of the syringe relative to the cassette unit.

WO17219157A1 shows a method for fixating a medicament cartridge in a cartridge holder of an injection device comprising at least one elastic attachment means that rotationally and axially fix the cartridge to the cartridge holder, wherein the fixture is such that axial and lateral movements, perpendicular to the longitudinal axis of the cartridge, are dampened or restricted.

Previous solutions for protecting the cartridge from breakage have focused mainly on absorbing axial forces (forces essentially parallel to a longitudinal axis of the cartridge) and require additional or extra parts, for example between a cartridge holder and the cartridge, thereby increasing complexity and cost of manufacturing. Furthermore, extra parts for protecting the cartridge take up additional space in the housing and thus lead to larger, less ergonomical and less practical devices. Additionally, many of the solutions found in prior art comprise a plastic deformation of the shock absorbing element and can thus effectively only prevent damage once.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device in which the plunger of the medicament moves during a delivery process, whereas the term "proximal" is meant to refer to the opposite direction or end. The longitudinal axis of the cartridge is referred to as L and extends along the center of the length of the cartridge in the

The term "injection system" or "injector" refers to a device that is removed from the injection site after each medication event or drug delivery process, whereas the term "infusion system" refers to a device with a cannula or needle that remains in the skin of the patient for a prolonged period, for example, several hours.

### DESCRIPTION OF THE INVENTION

The invention pertains to medicament delivery devices, particularly to a subassembly for a medicament delivery device adapted to hold or carry a medicament cartridge, wherein the medicament delivery device is preferably an on-body or patch injection device or an infusion pump. It is a first, generic objective of the invention to prevent cracking or breaking of the medicament cartridge inside of the medicament delivery device, as in case of drops or rough handling during transportation. According to ISO standard ISO 11608-1:2022, on-body injection devices must be able to withstand a drop from approximately 1m height without damage.

The invention departs from a medicament delivery device in which the cartridge is placed in close vicinity to a predominantly flat or planar housing section surface on the inside of the device, with no shock-absorbing extra component arranged between the outer surface of the cartridge and the housing. The opposite or outer surface of said housing section is an outside surface of the medicament delivery device, which means that there are no parts provided in between the location of impact on the housing and the cartridge in case of a drop in this direction. Intermediate parts between the cartridge and the location of impact are space-consuming but may be able to move relative to each other and to elastically or plastically deform, while absorbing kinetic energy from the fall and thus preventing breakage of the cartridge. On the other hand, directly mounting the cartridge on a housing wall allows for a space saving arrangement and leads to a compact and ergonomic device.

According to the above, an ideal cartridge holder in a medicament delivery device minimizes the use of additional parts while providing the ability to absorb high impact forces. Ideally, additional intermediate parts may be able to elastically deform and subsequently return to their initial position, maintaining functionality and structural integrity of the device. It is thus a further, refined objective of the invention to provide a space-saving and shock absorbing cartridge holder for a medicament delivery device that can efficiently absorb shocks or impacts by temporary elastic deformation while maintaining the functionality and compact geometry of the device.

This objective is achieved by providing a shock-absorbing cartridge holder in a medicament delivery device, the cartridge holder comprising at least two elastic members or shock absorbing elements that can elastically deform upon impact of the device and thus absorb the kinetic energy and brake or dampen a movement of the cartridge towards a device housing that could otherwise lead to damage. The shock absorbing elements are provided in the form of at least two parallel elastic supporting walls or fins which laterally (parallel and off-centered with respect to the longitudinal axis (L) of the cartridge) and vertically (perpendicular to the longitudinal axis (L)) support or carry the cartridge and can bend to the side (away from each other) in case the cartridge experiences an acceleration in a direction of the shock absorbing elements. Parallelity in this context means that the planes containing the walls are parallel to each other in an unstrained or initial state. The walls perpendicularly extend from a surface of a housing part or a portion of a housing part that is substantially flat or planar and is, in particular, not curved. This means that the majority of the surface is contained in a plane and does not exclude the presence of protrusions or recesses that may be present on the surface of the housing part. The elastic walls are combined with a recess in the housing, located between the walls, which provides a space for the cartridge to move towards the wall without taking damage. The cartridge or part of the cartridge may move or dive into said recess during the braking movement, wherein the dimensions and flexibility of the elastic walls is chosen such that the cartridge cannot touch the bottom of the recess or any other part of the housing surface. The shock absorbing elements or walls together with the recess make up a shock absorbing cartridge carrier or holder.

In case of sudden high forces on the cartridge, leading to an acceleration of the cartridge towards other parts of the medicament delivery device, particularly towards the housing part comprising the shock absorbing cartridge holder, the walls can elastically deform in a swiveling or bending motion thus braking or slowing the motion of the cartridge. In one embodiment of a medicament delivery device according to the invention the elastic walls or fins are mounted on an inner surface of the device housing that surrounds the cartridge. The elastic walls may be mounted to the surface via an adhesive connection or may be affixed to the wall via any other suitable means such as a press fit or a snap connection. The wall of the housing as well as the elastic fins or walls may be injection molded, wherein different plastics, preferably a harder plastic for the wall of the housing and a softer plastic for the elastic walls is used (2K injection molding). The elastic shock absorbing walls are accompanied by a recess in the housing, which provides space for the cartridge to move into or towards the housing surface during the braking motion. The recess is thus preferably located between the elastic walls supporting the cartridge and is preferably formed in a direction which is normal or perpendicular to the direction of the bending or swiveling motion of the elastic walls.

The shock absorbing cartridge holder according to the invention allows for a space efficient design of the medicament delivery device, leveraging the lateral support structure of the cartridge as shock absorbing element and using a recess in a housing part of the device as braking space, leading to a highly compact and space efficient solution.

The medicament cartridge insertable into or mountable in the medicament delivery device is preferably made from glass but may be made of any other material which is suitable to store or contain the medicament for a prolonged period of time. Preferably the medicament cartridge comprises a rubber septum, pierceable by a needle, and a metal crimp at a tapered distal end. The inner volume of the cartridge is proximally sealed by a rubber plunger or stopper that can be moved in a distal dispensing direction, causing the medicament to be forced through a needle attachable to the distal end of the cartridge. Such medicament cartridges usually comprise a rim with sharp edges at a proximal end, which can be susceptible to formation of cracks in case of impacts. Consequently, cracking of these glass cartridges most often starts from said rim and propagates in distal direction. It is thus of special importance to protect the proximal end of such a cartridge from high force impacts.

When the cartridge is mounted on the walls the shock absorbing walls thus preferably extend from a proximal end of the cartridge in the distal direction, parallel to a longitudinal axis of the cartridge, such that a proximal end of the elastic walls is at least approximately aligned with a proximal end of the cartridge. The walls can extend over the entire length of the cartridge or only along part of the cartridge, wherein the distal end can be supported by other holding means that do not need to be elastic or flexible. This allows the needle piercing the cartridge to safely do so without risk of damage to the septum or the needle in case of shifts of the cartridge relative to the needle due to elasticity of the distal holding element. The cartridge is thus preferably held fix at a distal end and can move laterally at a proximal end, wherein said lateral movement is enabled by the recess in the housing and mitigated or slowed by the elastic walls. The cartridge or, more precisely, the longitudinal axis (L) of the cartridge may be parallel to the surface of the device where the elastic walls are mounted in an initial or shipping state and may tilt during an impact or a braking movement. In particular, the proximal end may move closer to said surface.

The recess in the housing has a shape or outline and a form that follows the above-described motion of the cartridge. In particular, the recess is located between the elastic walls and extends from a distal portion of the cartridge to at least the proximal end of the cartridge, such that a movement of the proximal end portion of the cartridge into or towards the recess is enabled. The recess can have a uniform or continuous depth or can be slanted or shallow at one end and deeper at another end. In a preferred embodiment, the recess is shallow at a distal end thereof, and gradually becomes deeper towards the proximal end and comprises a proximal end section with uniform depth. The distance between the elastic walls and the recess is chosen in a way that the energy acting on the cartridge in case of impacts is minimized. The optimal distance between the recess and the walls, for which said energy is minimal, as well as the height of the wall and the depth of the recess may be determined by experiments or derived from simulations.

The housing of a medicament delivery device according to the invention, or at least the housing part comprising the recess is preferably made from a different material than the elastic walls. The device housing is preferably made from a hard material for protection of the mechanics and the medicament container, wherein the elastic walls are made from a relatively softer material, with the necessary elastic properties. Consequently, the Young's modulus of the housing material may be greater than the corresponding modulus of the elastic walls.

The medicament delivery device, or the parts making up the medicament delivery device, especially the parts making up the housing, are preferably produced by injection molding. The parts may be made from any suitable plastic material, such as polypropylene (PP) or polyoxymethylene (POM). Parts of the device that are subjected to high stress or high loads may be made from glass fiber reinforced plastic (GRP). The elastic walls are made from an elastic plastic material, preferably a thermoplastic elastomer or thermoplastic rubber (TPR) such as a thermoelastic polyamine or a thermoelastic polyurethane. The parts may be produced using 2K molding wherein in a first step, the device housing part on which the elastic walls are to be mounted is formed and in a second step the elastic walls are molded directly on the housing part.

In a preferred embodiment of a medicament delivery device according to the invention, the elastic walls and the housing parts on which said walls are to be mounted or fixed are produced separately and are assembled in a subsequent step, wherein the material for the elastic walls is softer than the material for the housing parts. The elastic walls may be affixed to the housing by any suitable means known in the art. In a preferred embodiment, the walls comprise cylindrical studs or pins that can be inserted into corresponding cylindrical bores in the device housing.

The medicament delivery device containing the shock absorbing element according to the present invention may be an injection or infusion device, adapted to dispense or release a dose of medicament from a cartridge through a needle or canula into the body of a patient or user. Preferably the dose of medicament is injected or released into the subcutaneous tissue of the patient or user. The device may further compromise mechanical or electromechanical contraptions for inserting the needle into or through the skin of a patient or user and a drive train to drive the plunger in the cartridge in distal direction in order to dispense the medicament from the cartridge. Furthermore, the device may comprise one or more human machine interfaces (HMI) such as in the form of buttons, displays, light signals or any other suitable means. The device may further comprise a needle protecting or shielding element that protects the sharp or tip of the needle before and/or after dispensing to prevent accidental injuries.

In a preferred embodiment of a medicament delivery device according to the invention, the shock absorbing cartridge carrier is a subassembly of a patch injection or infusion device which can be removably attached to a patient or user's body via an adhesive surface of the device and which comprises a needle insertable into the user's subcutaneous tissue to inject a medicament therethrough. The device may be adapted to dispense the entire content of the medicament cartridge at once or alternatively, the dispensing might be in discrete steps. The device may be left on the skin of the user for a short amount of time, preferably less than 30 minutes, wherein the entire content of the cartridge is dispensed at once or the device may be left on the skin of the user for a prolonged period of time, preferably multiple hours, wherein the contents of the cartridge are dispensed in discrete steps or intervals. The medicament is contained in a vitreous cartridge which has a substantially cylindrical shape. The cartridge comprises a rubber bung or stopper, proximally confining the inner volume, which is moveable towards the tapered distal end of the cartridge for a dispensing or delivery operation. The tapered distal end is sealed by a rubber septum which is pierceable by a needle. The septum may additionally be fixed to the cartridge by a metal or plastic crimp.

The recess in the housing is shaped in a way that allows a braking motion of the cartridge when the proximally located elastic walls swivel to the side, without the cartridge encountering the bottom of the recess. A braking motion of the cartridge into the recess is thus enabled. The recess extends from a distal portion of the cartridge, where the cartridge is non-elastically supported, to a proximal end of the cartridge. To allow some room for movement and manufacturing tolerances, the recess ideally extends beyond a proximal end, e.g. by a margin of at least a few millimeters.

The width of the recess is similarly chosen to be as wide as required for preventing the cartridge from encountering the bottom or the side walls when the elastic walls are bent to the side. As the cartridge will tilt during the braking movement, i.e. will move towards the housing part comprising the recess at the proximal end, a larger diameter of the recess will be required at the proximal end of the recess, compared to the distal end of the recess. The recess is thus shaped such that its diameter or width perpendicular to the longitudinal axis (L) gradually increases in the proximal direction.

The cartridge holder is designed such that only a bottom or peripheral part of the cartridge moves towards or into the recess during the braking motion. Thus, the maximum width of the recess does never exceed a maximum diameter of the cartridge, wherein said diameter of the cartridge is measured perpendicular to the longitudinal axis.

The depth of the recess is further shaped in a way that is adapted to the tilting braking movement of the cartridge during braking. As only the proximal end of the cartridge can move into or towards the recess, the recess is shallow at its distal end and gradually becomes deeper towards the proximal end. This provides the necessary space for the braking movement while maintaining structural integrity of the housing.

In a preferred embodiment of a medicament delivery device comprising a cartridge holder according to the invention, the cartridge holder is part of a patch injection or infusion device which is adapted to be adhesively attached to the body of a user for a prolonged period of time, i.e. for a period of more than one minute, preferably more than 5 minutes. The medicament may either be continuously dispensed or may be dispensed in discrete time intervals of variable length.

The patch injection or infusion device may further comprise:
- a drive mechanism, adapted to move the plunger of the cartridge into a distal dispensing direction
- an injection needle, adapted to be inserted into the skin, more precisely into the subcutaneous tissue of the user
- a cartridge needle, adapted to pierce a septum at the distal end of the cartridge and thereby establishing a fluid path between the inner volume of the cartridge and the injection needle, such that the cartridge contents can be dispensed into the subcutaneous tissue of the user
- a needle insertion mechanism, adapted to cause said injection needle to pierce said septum and to cause said injection needle to pierce said skin.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of s first embodiment of an injection device comprising a cartridge holder according to the present invention;
- Fig.2: depicts a section view along the longitudinal axis (L) of the cartridge of a second embodiment of an injection device comprising a cartridge holder according to the present invention
- Fig.3: depicts a section view perpendicular to the longitudinal axis (L) of the device of figure 1
- Fig.4: depicts a section view perpendicular to the longitudinal axis (L) of the device of figure 1
- Fig.5: depicts a perspective view of the housing base of the device of figure 1

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

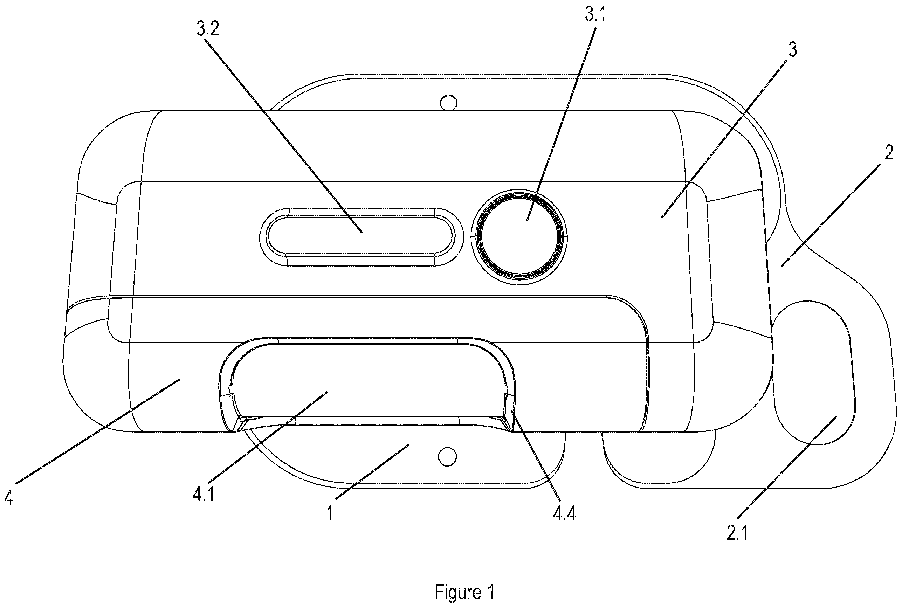

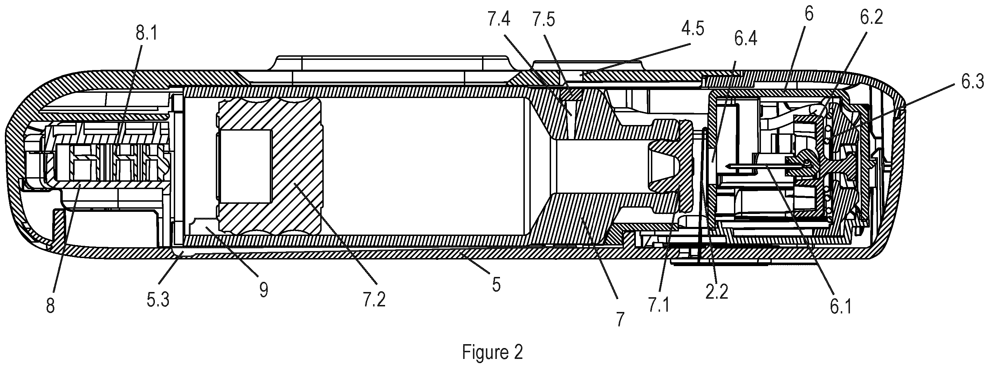

In figure 1, a medicament delivery device comprising a cartridge holder according to the present invention is depicted. The device comprises an adhesive patch 1, which, in a delivery state, is covered by a release liner 2 that must be removed by the user before the device can be attached to the skin. A pull tab 2.1 can be grabbed by the user and facilitates removal of the release liner. A housing lid 3 covers a drive mechanism unit and a needle insertion mechanism unit, as well as a medicament cartridge and additional components, which are mounted on a housing base 5 (visible in figure 2) The drive mechanism is adapted to move a plunger in the medicament cartridge in distal direction for dispensing of medicament and may also be adapted to drive and control the needle insertion mechanism. A window 4.1, whose outline is defined by a cut out 4.2 in the cartridge lid, provides visual access to the medicament cartridge and may be used to visually check the contents and to track progress of a delivery process. A start button 3.1 is pressed by the user to initiate a delivery process, which delivery process can be monitored by a status bar 3.2. The status bar is partially transparent and is illuminated by an array of LED lights mounted on a PCB board underneath the housing lid 3. Start button 3.1 is operably connected to a switch on the PCB board, which is adapted to control the drive and insertion mechanisms.

Figure 2 shows a section view of a second embodiment of a medicament delivery comprising a cartridge holder according to the present invention, wherein the section plane is along the longitudinal axis of the medicament cartridge and wherein the adhesive patch 1 and the release liner 2 are omitted. A needle insertion unit 6 is mounted on housing base 5 and comprises a cartridge needle 6.1 which is adapted to pierce a septum 7.1 of cartridge 7 and thereby establish a fluid path to the injection needle. To this end, the cartridge needle is mounted on a cartridge needle slider 6.2, which is biased in the distal direction by the cartridge needle spring 6.3, which cartridge needle spring can be released by pushing the button 3.1. Opening 6.4 of needle insertion unit 6 is initially covered by a sterile barrier film 2.2 which is connected to and removed together with the release liner. Proximally of the cartridge, a segmented plunger rod 8.1 is arranged, forming part of a drive unit 8 and being adapted to move a plunger 7.2 in distal direction in the cartridge after a medicament delivery process has been initiated. The cartridge holder according to the invention is visible near the proximal end of the cartridge 7 and comprises a recess 5.3 in housing base 5 and elastic walls 9. Elastic walls 9 and 9.1 are mounted on the housing base 5 and support the cartridge laterally. Recess 5.3 extends from a distal portion cartridge beyond the proximal end of the cartridge. The elastic walls 9 and 9.1 (not shown) extend from a proximal portion of the cartridge in the distal direction and have a length along the longitudinal axis of about one fifth of the total cartridge length. The depth of recess 5.3 is such that the cartridge never touches the bottom of the recess, even when the elastic walls are maximally elastically deflected.

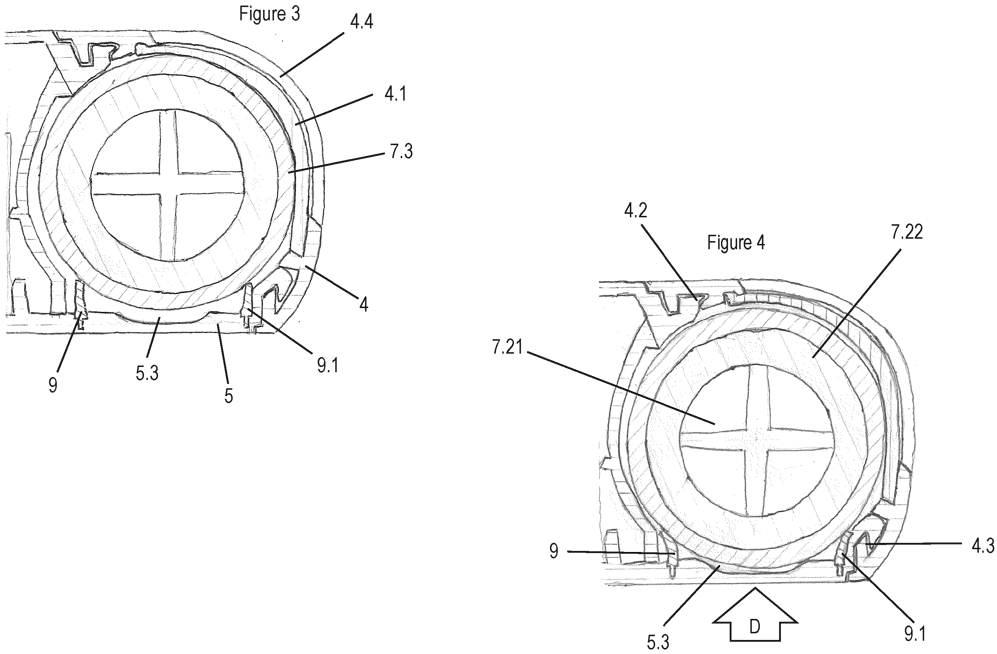

Figure 3 depicts a cross section of a medicament delivery device comprising the cartridge holder according to the invention in an initial or delivery state and at rest. The section plane extends perpendicular to the longitudinal axis (L) and through a proximal portion of the cartridge. The space containing the cartridge is closed off by cartridge lid 4, comprising window 4.1. Cartridge lid 4 is fixed to housing base 5 and housing lid 3 via snap fit connections 4.2 and 4.3. On the opposite side of the cartridge, a wall 6.4 of the needle insertion unit extends coaxially along the length of the cartridge. The cartridge has an outer glass wall 7.3 with an annular cross section which is in contact with a rubber seal 7.22 surrounding a plastic plunger core 7.21, wherein a sealing gap is formed between the glass wall and the rubber seal. The cartridge is carried on two elastic walls 9 and 9.1 which are mounted on housing bottom 5 and extend coaxially to the cartridge perpendicular to said housing bottom. Elastic walls 9, 9.1 are mounted on the housing base bottom wall 9 via pins 9.2 that extend into bores 5.2 in bottom wall. This method of mounting is not to be construed as scope limiting, other methods are laid out in the general description.

Figure 4 depicts a cross section of a medicament delivery device comprising the cartridge holder according to the invention. In this section view the device is shown during an impact from direction D, leading to an acceleration of the device housing in direction D relative to the cartridge and a consequential elastic deformation or bend of the elastic walls away from the longitudinal axis (L) of the cartridge. The proximal part of the cartridge has moved in a direction opposite to D relative to the housing and is now partially located in recess 5.3.

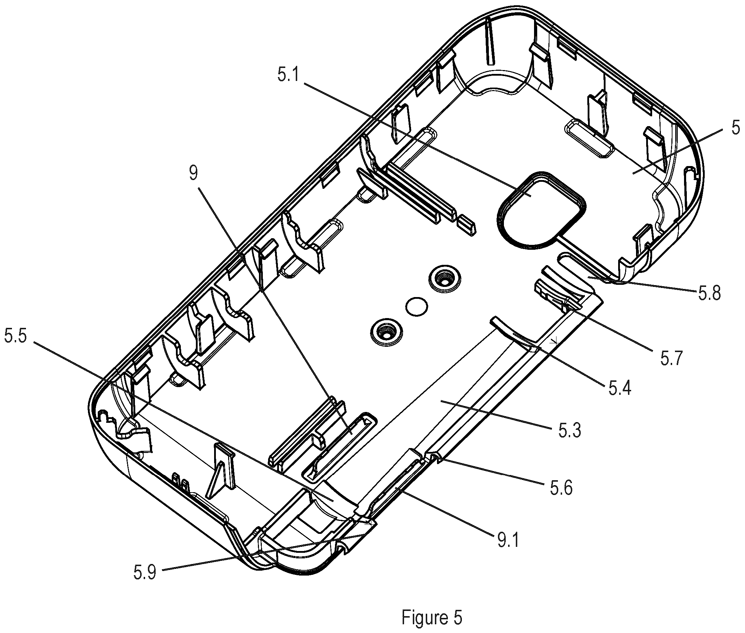

Figure 5 depicts a perspective view of housing base 5 of a preferred embodiment of a medicament delivery device according to the invention. A central cutout 5.1 is provided for the injection needle (not shown) to pass through, which is sealed by a sterile barrier in the delivery state. A further cutout 5.8 provides a passage for a second sterile barrier 2.2 (visible in figure 2) which seals the cartridge in an initial state and is removed together with release liner 2 before use. An elongated latch 5.9 extends along one edge of housing base 5 and is adapted to form a snap fit connection with cartridge lid 4. The ledge is interrupted by a cut out 5.6 which provides space for the second elastic wall 9.1 of the cartridge holder. The cartridge holder further comprises a first elastic wall 9 and recess 5.3 which gradually becomes deeper in the proximal direction and terminates in a bottom section 5.5 of uniform depth. At the proximal end of the recess, an axial support wall 5.4 fixates the cartridge in the axial (L) direction wherein a second support wall 5.7 supports the cartridge at the proximal end.

While the invention has been described in detail in the drawings and foregoing description, such description is to be considered illustrative or exemplary and not restrictive. Variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- 1: adhesive patch
- 2: release liner
- 2.1: pull tab
- 2.2: sterile barrier
- 3: housing lid
- 3.1: button
- 3.2: status bar
- 4: cartridge lid
- 4.1: window
- 4.2: first snap fit connection
- 4.3: second snap fit connection
- 4.4: cartridge lid cut out
- 4.5: fill port hole
- 5: housing base
- 5.1: needle cut out
- 5.3: recess
- 5.5: recess proximal end part
- 6: needle insertion unit
- 6.1: cartridge needle
- 6.2: cartridge needle slider
- 6.3: cartridge needle spring
- 6.4: needle insertion unit opening
- 7: cartridge
- 7.1: septum
- 7.2: plunger
- 7.21: plunger core
- 7.22: rubber seal
- 7.3: glass wall
- 7.4: fill port
- 7.5: fill port seal
- 8: drive unit
- 8.1: segmented plunger rod
- 9: first elastic wall
- 9.1: second elastic wall

## Claims

1. A cartridge holder in a medicament delivery device with a housing, wherein the cartridge holder comprises at least two walls protruding perpendicularly from a substantially planar inner surface of the housing and wherein the walls are adapted to support a cartridge mountable in the device, **characterized in that**:
a) the walls are extending parallel to a longitudinal axis (L) of a mounted medicament cartridge,
b) the cartridge holder comprises a recess, located between the at least two walls in the inner surface of the housing; and
c) the at least two walls can elastically bend away from the recess, allowing the cartridge to move towards the recess.

2. The cartridge holder according to claim 1, wherein a proximal end of the elastic walls is at least approximately axially aligned with a proximal end of the cartridge.

3. The cartridge holder according to claim 1 or 2, wherein the housing and the at least two elastic walls are made from different plastic materials.

4. The cartridge holder according to claim 3, wherein the housing and the at least two elastic walls are produced by 2K injection molding.

5. The cartridge holder according to claim 3 or 4, wherein the at least two elastic walls are made from a thermoplastic elastomer (TPE).

6. The cartridge holder according to any of the previous claims, wherein the cartridge is a vitreous cartridge comprising a pierceable septum and optionally a metal crimp at a distal end.

7. The cartridge holder according to any of the previous claims, wherein the recess in the inner housing surface extends from a distal portion of the cartridge to the proximal end of the cartridge, such that a movement of a proximal part of the cartridge into the recess is enabled

8. The cartridge holder according to any of the previous claims, wherein a width of the recess measured perpendicular to the longitudinal axis (L) gradually increases in the proximal direction.

9. The cartridge holder according to claim 8, wherein the width of the recess does not exceed a maximum width of the cartridge when measured perpendicular to the cartridge.

10. The cartridge holder according to any of the previous claims, wherein the recess in the housing is shallow at its distal end and gradually becomes deeper towards its proximal end.

11. Medicament delivery device comprising the cartridge holder of any of the previous claims, wherein, for a medicament delivery process, the medicament delivery device is adapted to be adhesively attached to the skin of a user for a time of at least 5 minutes.

12. Medicament delivery device according to claim 11, wherein the medicament delivery device further comprises
- an injection needle, adapted to be inserted into the skin of a user,
- a cartridge needle, adapted to be inserted into the medicament cartridge,
wherein the injection needle and the cartridge needle are connected by a fluid path inside the injection device and are at least approximately orthogonally oriented relative to each other.
